# EUROPÄISCHE PATENTSCHRIFT

(11) **EP 0 423 431 B1**
(45) Veröffentlichungstag und Bekanntmachung des Hinweises auf die Patenterteilung: **21.04.1993**
(21) Anmeldenummer: 90113205.0
(22) Anmeldetag: 11.07.1990
(51) Int. Cl.: B23K 26/00, A61B 17/36

(54) **Chirurgisches Laserinstrument**
Surgical laser
Laser chirurgical

(30) Priorität: 17.10.1989 DE 3934647
(43) Veröffentlichungstag der Anmeldung: 24.04.1991
(73) Patentinhaber: Deutsche Aerospace AG, 81663 München (DE)
(72) Erfinder: Hessel, Stefan, Dr., D-8000 München 81 (DE); Hauptmann, Gerhard, D-8000 München 82 (DE)

(56) Entgegenhaltungen:
- EP-A- 0 212 786
- EP-A- 0 245 552
- WO-A-88/08279
- US-A- 4 718 417

## Beschreibung

Die Erfindung betrifft ein chirurgisches Laserinstrument zum wechselweisen Kontaktschneiden und kontaktlosen Koagulieren von biologischem Gewebe mittels einer faseroptisch geführten Strahlung eines Lasers, welcher hierzu mit einem faseroptischen Lichtleiter als Schneid- und Koagulationswerkzeug verbunden ist, und mit einem Strahlungsdetektor, auf den die über den faseroptischen Lichtleiter rückgeführte und ausgefilterte Strahlung gerichtet wird (sieh EP-A-245552).

In der Laserchirurgie wird in vielen Fällen das Kontaktschneiden dem kontaktlosen Schneiden vorgezogen, da es schwierig ist, das faseroptische Instrument in einem gleichmäßigen Abstand über dem Gewebe zu führen. Hinzu kommt, daß bei den bisherigen Lasersystemen unbedingt vermieden werden muß, die Lichtleitfaser während der Bestrahlung mit dem Gewebe in Kontakt zu bringen, da sonst sofort die auf der Faser haftenden Geweberückstände in die Faser einbrennen und letzlich zu einer Überhitzung und Bruch der Faser führen. Aus diesem Grund wird in der US-PS 4 693 244 ein chirurgisches Laserinstrument vorgeschlagen, welches zum Zwecke des Kontaktschneidens am Ende einer Lichtleitfaser eine Saphirspitze trägt, welche die thermischen Belastungen beim Kontaktschneiden besser verträgt. Diese in der Laserchirurgie bereits eingeführten Saphirspitzen sind relativ teuer und zweckmäßiger Weise auch nur zum Schneiden geeignet Zudem ist auch die Standzeit derartiger Saphirspitzen begrenzt und vermindert sich drastisch bei nicht vorschriftsmäßiger Handhabung. Schließlich ist eine gerichtete Koagulation unter ständiger optischer Kontrolle des jeweils bestrahlten Gewebeflecks damit nur schwer möglich.

Auch bei dem in der EP 0 245 552 beschriebenen Laserkatheter wird ein Spahirelement an der Spitze des Lichtleiters verwendet, jedoch wird hier die im Saphir durch die Laserstrahlung angeregte Fluoreszenzstrahlung dazu benutzt, um den Laser ggf. abzuschalten. Ein Schneiden mit der blanken Faser ist also nicht möglich, zumal keine Leistungsregelung des Lasers während der Bestrahlung vorgesehen ist.

Es ist daher Aufgabe der Erfindung ein chirurgisches Laserinstrument zum wechselweisen Kontaktschneiden und kontaktlosen Koagulieren von biologischem Gewebe mittels einer faseroptisch geführten Strahlung eines Lasers zu schaffen, welches bei gleichbleibend hohen sicherheitstechnischen Anforderungen, insbesondere hinsichtlich Standzeit des faseroptischen Lichtleiters, ein vielfältigeres Arbeiten als mit dem bekannten Instrument ermöglicht.

Diese Aufgabe wird durch ein nach den Merkmalen des Patentanspruchs 1 ausgebildetes chirurgisches Laserinstrument gelöst.

Die Erfindung beschreibt einen anderen Weg, als er durch die oben erwähnte US-PS 4 693 244 aufgezeigt wurde. Ein Schutz des faseroptischen Lichtleiters, insbesondere beim Kontaktschneiden, läßt sich erfindungsgemäß dadurch erreichen, daß das bei der Verbrennung von biologischem Gewebe im sichtbaren Spektralbereich zwischen 0,3 und 0,9 µm ("Weißlicht") emittierte Licht erfaßt und zur Leistungsregelung des Lasers verwendet wird. Unter dem Begriff faseroptischer Lichtleiter werden hier sämtliche Komponenten verstanden, die das Laserlicht zu dem zu behandelnden Gewebe führen, so daß dieser Schutz sowohl bei Verwendung der bloßen Faser als Schneid- und Koagulationswerkzeug, als auch für solche faseroptischen Lichtleiter gilt, bei denen ein Applikator aus zumindest teilweise optisch transparentem Material, z.B. eine Saphirspitze, an das distale Ende der Lichtleitfaser angekoppelt ist.

Aus der DE 38 13 918 Al ist zwar eine Vorrichtung zur Laserbehandlung von Gewebe bekannt, bei welchem ein Sensor die von dem zu behandelnden Gewebe aufgrund der auftreffenden Laserstrahlung ausgehende Fluoreszenzstrahlung erfaßt und in Verbindung mit einer Spektralanalyseeinheit eine Identifizierung des Gewebes ermöglicht. Aufgrund dieser Informationen läßt sich die Laserbestrahlung optimieren. Die der vorliegenden Erfindung zugrundeliegende Aufgabe läßt sich jedoch damit nicht lösen, zumal die vom Gewebe emittierte Fluoreszenzstrahlung keine eindeutige Aussage über die thermische Belastung des Lichtleiters zuläßt. Da die erfindungsgemäß vorgesehenen Maßnahmen sowohl den Karbonisierungsprozeß des biologischen Gewebes als auch die unmittelbare thermische Belastung der Faser bei Verbrennung der auf dem distalen Ende der Faser haftenden Geweberückstände erfaßt, wird durch die vorgesehene Regelung der Laserleistung sichergestellt, daß auch bei anhaftenden Geweberückständen am Faserende die Zerstörungsschwelle des Lichtleitermaterials nicht überschritten wird und daß aufgrund dessen ein gleichmäßiges Kontaktschneiden im Gewebe möglich wird. Sollen nach dem Kontaktschneiden bestimmte Gewebepartien kontaktlos koaguliert werden, so genügt es, den durch Geweberückstände verschmutzten Lichtleiter aus dem Schnitt herauszuziehen und in einem gut zu beobachtenden Abstand vor die zu koagulierende Gewebestelle zu halten. Da nunmehr die bisher vornehmlich vom zu schneidenden Gewebe emittierte Strahlung fehlt, wird die Strahlungsleistung des Lasers solange erhöht, bis die Geweberückstände auf dem Lichtleiter verbrennen und die dabei entstehende Strahlung einen vorgegebenen Wert, der die Zerstörungsschwelle des Lichtleiters repräsentiert, erreicht. Das Faserende wird dann zunehmend transparenter für die Laserstrahlung, so daß der Laser bis an eine vorgegebene Leistungsgrenze hochgeregelt wird und dann zum Koagulieren bei ausreichendem Abstand zwischen Faserende und Gewebe eingesetzt werden kann. Die Erfindung löst somit in überraschender Weise gleich mehrere Probleme, so daß, anders als bisher, sogar mit der blanken Faser sowohl wechselweise ein Kontaktschneiden als auch ein kontaktloses Koagulieren mit höherer Standzeit als bisher möglich wird.

Die Erfindung wird im folgenden anhand der in den Figuren teilweise schematisch dargestellten Ausführungsbeispiele näher beschrieben. Es zeigen:
Fig. 1 ein chirurgisches Laserinstrument zu Freihandarbeiten und
Fig. 2 ein chirurgisches Laserinstrument zu endoskopischen Zwecken.

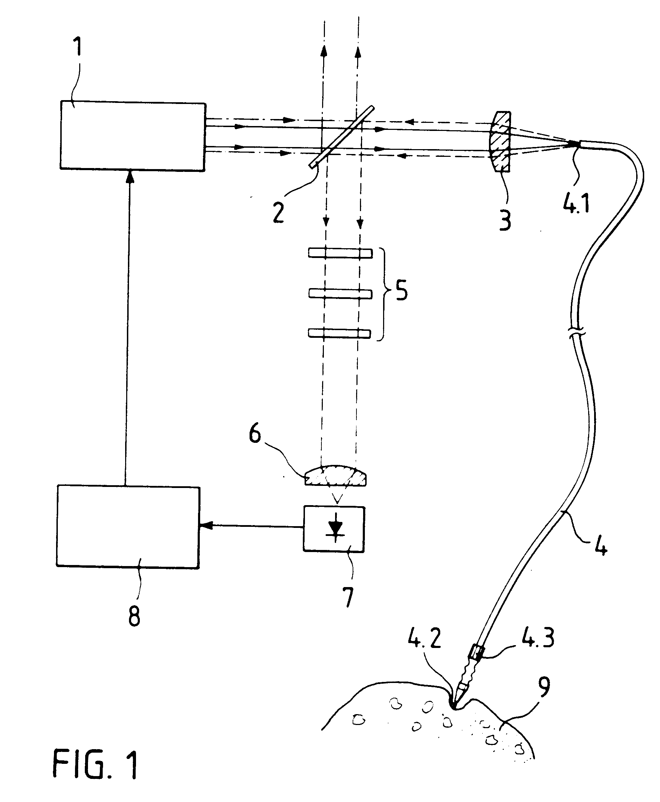

Bei dem in Fig. 1 dargestellten Ausführungsbeispiele wird die Strahlung eines medizinischen Lasergerätes 1 über einen wellenlängenselektiven Strahlteiler 2 und eine Optik 3 in das proximale Ende 4.1 einer Lichtleitfaser 4 eingekoppelt. Das Lasergerät 1 ist ein Neodym-YAG-Therapielaser, dessen Strahlung bei etwa 1 µm, also im nahen Infrarotbereich liegt. Der Strahlteiler 2 ist als wellenlängenselektiver Spiegel ausgebildet, wobei die Beschichtungen derart ausgewählt werden, daß die Strahlung des Neodym-YAG-Lasers ungehindert passieren kann, jedoch sowohl die laserseitig von den Pumplichtquellen, vor allem im sichtbaren Bereich abgegebene Strahlung, als auch die lichtleiterseitig zurückgeführte Strahlung im Spektralbereich zwischen 0,3 und 0,9 µm reflektiert werden. Während die laserseitig reflektierte Strahlung ungenutzt abgeleitet bzw. absorbiert wird, gelangt die lichtleiterseitig reflektierte Strahlung über ein Filtersystem 5 und eine weitere Optik 6 auf einen Strahlungsdetektor 7, dessen Ausgangssignal in einer elektronischen Regelschaltung 8 ausgewertet und zur Steuerung des Lasers 1 verwendet wird. Letzteres kann beispielsweise durch Regulierung der Stromversorgung für die Pumplichtquelle geschehen.

Die in die Lichtleitfaser 4 eingekoppelte Therapiestrahlung wird auf das zu behandelnde Gewebe 9 gerichtet, wozu das distale Ende des Lichtleiters von einem Handstück 4.3 umgeben ist. Aus diesem Handstück 4.3 ragt das distale Ende 4.2 der Lichtleitfaser einige Millimeter heraus und ist von seiner Ummantelung (Buffer) im Bereich der äußersten Spitze befreit.

Die beim Karbonisierungsprozeß des bestrahlten Gewebes emittierte Strahlung, die den wesentlichen Bereich des sichtbaren Spektrums mit umfaßt, wird vom distalen Ende 4.2 der Lichtleitfaser empfangen, über diese zurückgeleitet und trifft auf den Strahlteiler 2, an dessen wellenlängenselektiv wirkenden Schicht eine Ablenkung der Strahlung aus dem Strahlengang des Lasers 1 heraus in Richtung Strahlungsdetektor 7 erfolgt. Zur Begrenzung der empfangenen Strahlung auf den sogenannten Weißlichtbereich ist das optische Filter 5 als Bandpaßfilter für den Bereich zwischen 0,3 und 0,9 µm, vorzugsweise zwischen 0,4 und 0,8 µm ausgelegt. Die Dämpfung des Filters sollte dabei für die zurückzuhaltenen Spektralbereiche besser als 10⁵ sein.

Die Regelung der Laserleistung aufgrund des Detektorsignals erfolgt derart, daß ein vorgebbarer Wert der empfangenen Strahlungsleistung im Detektor 7, welcher die Zerstörungsschwelle des jeweils verwendeten Lichtleiters repräsentiert, nicht überschritten wird.

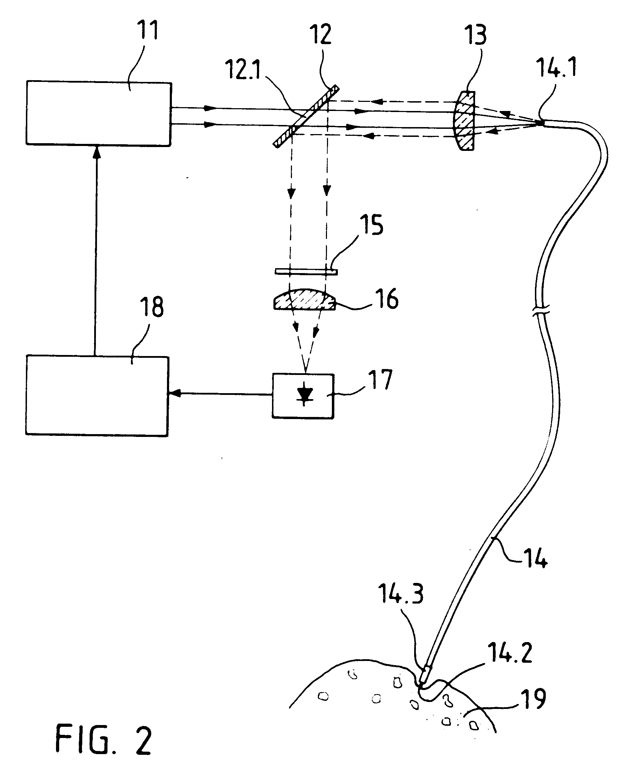

Das Ausführungsbeispiel gemäß Fig. 2 unterscheidet sich von dem vorhergehenden vor allem dadurch, daß es zu endoskopischen Zwecken verwendet wird; das distale Ende der Lichtleitfaser 14 trägt dazu einen im Röntgenlicht erkennbaren Überzug 14.3. Der Strahlteiler 12 zwischen dem Laser 11 und der Einkoppeloptik 13 besteht hier aus einem breitbandigen Reflektor, z.B. einem metallischen Spiegel, in dessen Mitte eine Öffnung 12.1 vorhanden ist, durch den die Therapiestrahlung des Lasers 11 ungehindert hindurchtreten und über die Optik 13 in die Lichtleitfaser 14 eingekoppelt werden kann. Das von den Verbrennungsprozessen des Gewebes 19 bei Laserbestrahlung emittierte Licht im Spektralbereich zwischen 0,3 und 0,9 µm wird wiederum vom distalen Ende 14.2 der Lichtleitfaser 14 empfangen, zurückgeleitet und am proximalen Ende 14.1 mit dem vollen Aperturwinkel der Faser abgestrahlt. Der über die Optik 13 erzeugte Strahlquerschnitt der rückgeführten Strahlung ist größer als derjenige der Laserstrahlung und trifft daher den Strahlteiler 12 auch außerhalb der Öffnung 12.1. Die rückgeführte Strahlung wird so an dem Strahlteiler 12 reflektiert und gelangt über ein Filter 15, welches den Spektralbereich der Laserstrahlung unterdrückt und eine Optik 16 auf einen Strahlungsdetektor 17, der analog zum vorhergehenden Ausführungsbeispiel ein Signal an einen Regler 18 abgibt, der seinerseits den Laser 11 bezüglich seiner Strahlungsleistung regelt.

Ein sehr heißer, in seiner Temperatur gesteuerter, sogenannter "Hot Tip" kann dadurch erzeugt werden, daß in die Kernschicht der Lichtleitfaser 14 an deren distalem Ende 14.2 ein Laserlicht absorbierendes Material, z.B. Kohlenstoff eingelagert ist, welches unter Luftabschluß lediglich aufglüht und dessen Strahlung über die Regelschleife auf einem konstanten Wert gehalten wird.

## Patentansprüche

1. Chirurgisches Laserinstrument zum wechselweisen Kontaktschneiden und kontaktlosen Koagulieren von biologischem Gewebe (9, 19) mittels einer faseroptisch geführten Strahlung eines Laser (1,11), welcher hierzu mit einem faseroptischen Lichtleiter (4, 14) als Schneid- und Koagulationswerkzeug verbunden ist, und mit einem Strahlungsdetektor (7, 17), auf den die über den faseroptischen Lichtleiter rückgeführte und, ausgefilterte Strahlung gerichtet wird, **gekennzeichnet** durch eine Bandpaßfiltereinrichtung (2,5; 12, 12.1, 15), der die von dem biologischen Gewebe (9,19) aufgrund von Karbonisationsprozessen emittierte, über das distale Ende (4.2, 14.2) des faseroptischen Lichtleiters (4, 14) wieder empfangene und über den faseroptischen Lichtleiter (4, 14) rückgeführte Strahlung in einem bandbegrenzten Spektralbereich zwischen 0,3 und 0,9 µm ausfiltert,
sowie
eine Einrichtung (8, 18) zur Regelung der Strahlungsleistung des Lasers (1, 11), die mit dem Strahlungsdetektor (7, 17) verbunden ist, wobei die Strahlungsleitung des Lasers (1, 11) mittels des Detektorausganssignals auf einen unter der Zerstörungsschwelle des faseroptischen Lichtleiters (4, 14) gelegenen Wert derart geregelt werden kann daß die bei Verbrennung sowohl des zu schneidenden biologischen Gewebes (9, 19) als auch der auf dem distalen Ende (4.2, 14.2) des faseroptischen Lichtleiters (4, 14) haftenden bzw. in diesen eingebrannten Geweberückstände emittierte Strahlung einen vorgegebenen Wert nich überschreitet.

2. Chirurgisches Laserinstrument nach Anspruch 1 **gekennzeichnet** durch einen Laser (1, 11), dessen Strahlung über eine Optik (3, 13) in das proximale Ende (4.1, 14.1) einer Lichtleitfaser (4, 14) eingekoppelt wird, einen im Strahlengang zwischen dem Laser (1, 11) und der Optikf (3, 13) angeordneten Strahlteiler (2, 12), durch den zum einen die Strahlung des Lasers (1, 11) ungehindert passiert und zum anderen die vom distalen Ende (4.2, 14.2) der Lichtleifaser (4, 14) empfangene, rückgeführte und aus dem proximalen Ende (4.1, 14.1) austretende Strahlung reflektiert und auf den Strahlungsdetektor (7, 17) gerichtet wird, wobei die reflektierende Fläche des Strahlteilers (2, 12) und/oder ein zwischen dem Strahlteiler (2, 12) und dem Strahlungsdetektor (7, 17) angeordnetes Filter (5, 15) außerhalb eines Spektralbereichs zwischen 0,3 und 0,9 µm sowie in demjenigen der Laserstrahlung eine Dämpfung besser 10⁵ aufweist und, daß das distale Ende (4.2, 14.2) der Lichtleitfaser (4, 14) von seiner Ummantelung befreit und zum Schneiden in oder Koagulieren über einem biologischen Gewebe (9, 19) führbar ist.

3. Chirurgisches Laserinstrument nach Anspruch 1 oder 2, **dadurch gekennzeichnet,** daß an dem distalen Ende (4.2, 14.2) der Lichtleitfaser (4, 14) in oder auf deren Kernschicht ein die Laserstrahlung zumindest teilweise absorbierendes Material eingelagert ist.

## Claims

1. Surgical laser instrument for alternate contact cutting and contact-free coagulation of biological tissue (9, 19) by means of fibre-optically guided radiation of a laser (1, 11) which is, for said purpose, connected to a fibre-optic light guide (4, 14) as cutting and coagulation tool, and with a radiation detector (7, 17), onto which is directed the radiation which has been returned via the fibre optic and filtered out, **characterised by** a bandpass-filtering arrangement (2,5; 12, 12.1, 15) which filters out radiation, which has been emitted by a biological tissue (9, 19) due to carbonizing processes, again received via a distal end (4.2, 14.2) of the fibre-optic light guide (4, 14) and returned via the fibre-optic light guide (4, 14), in a band-defined spectral range between 0̸.3 and 0̸.9 µm, and by
a device (8, 18) for controlling the radiation output of a laser (1, 11), which is connected to a radiation detector (7, 17), wherein the radiation output of a laser (1, 11) is controlled by means of a detector output signal to a value below a destruction threshold of the fibre-optic light guide (4) in such a manner that radiation emitted during combustion of both a biological tissue to be cut (9, 19) and of tissue residue adhering to or burnt into the distal end (4.2, 14.2) of a fibre-optic light guide (4, 14) does not exceed a specified value.

2. Surgical laser instrument according to claim 1, **characterised by** a laser (1, 11), the radiation of which is coupled via an optic (3, 13) into the proximal end (4.1, 14.1) of an optical fibre (4, 14), a beam divider (2, 12), which is arranged in the beam path between a laser (1, 11) and an optic (3, 13) and through which, on the one hand, radiation of a laser (1, 11) can pass unhindered and, on the other hand, radiation, which is receiZed by the distal end (4.2, 14.2) of the optical fibre (4, 14), returned and exiting from the proximal end (4.1, 14.1), is reflected and directed towards radiation detector (7, 17), in which respect the reflecting surface of the beam divider (2, 12) and/or a filter (5, 15) arranged between the beam divider (2, 12) and the beam detector (7, 17) has outside a spectral range between 0̸.3 and 0̸.9 µm as well as within that of a laser radiation a dampening better than 10̸⁵, and that the distal end (4.2, 14.2) of the optical fibre (4, 14) is released from its casing and guided for the purpose of cutting in, or coagulating over a biological tissue (9, 19).

3. Surgical laser instrument according to claim 1 or 2, **characterised in that** at the distal end (4.2, 14.2) of an optical fibre (4, 14), in or on its core surface, is placed an at least partially laser-radiation absorbing material.

## Revendications

1. Instrument chirurgical à laser permettant alternativement l'incision par contact et la coagulation sans contact de tissus biologiques (9, 19) au moyen d'un rayon émis par un laser (1, 11) et guidé par fibre optique, lequel laser est connecté à un conducteur de lumière (4, 14) à fibre optique faisant fonction d'instrument d'incision et de coagulation et comporte un détecteur de rayon (7, 17) sur lequel est dirigé le rayon filtré renvoyé par le conducteur de lumière à fibre optique, caractérisé par un système de filtre passe-bande (2, 5; 12, 12.1, 15) qui laisse passer le rayonnement qui est émis par le tissus biologique (9, 19) à la suite de processus de carbonisation, est capté par l'extrémité distale (4.2, 14.2) du conducteur de lumière (4, 14) à fibre optique et est renvoyé par le conducteur de lumière (4, 14) à fibre optique dans une zone spectrale limitée, comprise entre 0,3 et 0,9 µm, ainsi que par un dispositif (8, 18) de réglage de la puissance du laser (1, 11) qui est connecté au détecteur (7, 17), la puissance d'émission du laser (1, 11) pouvant être réglée à l'aide du signal de sortie du détecteur à une valeur inférieure au seuil de destruction du conducteur de lumière (4, 14) à fibre optique de manière telle, que le rayonnement émis lors de la combustion du tissus biologique (9, 19) à inciser et et de la combustion des résidus de tissus qui adhèrent sur l'extrémité distale (4.2, 14.2) du conducteur de lumière à fibre optique (4, 14) ou sont incrustés celle-ci ne dépassent pas une valeur prédéterminée.

2. Instrument chirurgical à laser selon la revendication 1, caractérisé par un laser (1, 11) dont le rayon est couplé au moyen d'une optique (3, 13) dans l'extrémité proximale (4.1, 14.1) d'une fibre optique (4, 14), un diviseur de rayon (2, 12) placé sur le trajet du rayon entre le laser (1, 11) et l'optique (3, 13), lequel diviseur d'une part laisse passer librement le rayon venant du laser (1, 11) et d'autre part réfléchit le rayon capté par l'extrémité distale (4.2, 14.2) de la fibre optique (4, 14) et sortant de l'extrémité proximale (4.1, 14.1) de celle-ci et le dirige sur le détecteur de rayon (7, 17), la surface réfléchissante du diviseur de rayon (2, 12) et/ou un filtre (5, 15) placé entre le diviseur de rayon (2, 12) et le détecteur de rayon (7, 17) présentant un affaiblissement supérieur à 10⁵ en dehors d'une zone spectrale comprise entre 0,3 et 0,9 µm et dans la plage spectrale du rayon laser, et par le fait que l'extrémité distale (4.2, 14.2) de la fibre optique (4, 14) est dégagée de sa gaine et peut être déplacée dans un tissus biologique (9, 19) pour inciser celui-ci ou sur le dit tissus pour le coaguler.

3. Instrument chirurgical à laser selon la revendication 1 ou 2, caractérisé par le fait qu'un matériau qui absorbe au moins partiellement le rayon laser est inséré dans ou est appliqué sur le coeur, à l'extrémité distale (4.2, 14.2) de la fibre optique (4, 14).
